(19)

Europäisches
Patentamt
European
Patent Office
Office européen
des brevets

(11) **EP 3 851 097 A1**

(12) **EUROPEAN PATENT APPLICATION**

(43) Date of publication:
**21.07.2021 Bulletin 2021/29**

(21) Application number: **20382021.2**

(22) Date of filing: **15.01.2020**

(51) Int Cl.:
**A61K 9/10** *(2006.01)* **A61K 9/14** *(2006.01)*
**A61K 47/02** *(2006.01)* **A61K 31/498** *(2006.01)*
**A61K 31/573** *(2006.01)* **A61K 9/00** *(2006.01)*

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
**KH MA MD TN**

(71) Applicants:
• **Universidad De Zaragoza**
  **50009 Zaragoza (ES)**
• **Fundación Instituto de Investigación Sanitaria
  Aragón (IIS Aragón)**
  **50009 Zaragoza (ES)**
• **Consejo Superior de Investigaciones Cientificas**
  **28006 Madrid (ES)**

(72) Inventors:
• **MAYORAL MURILLO, José Antonio**
  **50009 Zaragoza (ES)**

• **PABLO JULVEZ, Luis Emilio**
  **50009 Zaragoza (ES)**
• **POLO LLORENS, Vicente**
  **50009 Zaragoza (ES)**
• **VISPE PALACÍN, Eugenio**
  **50009 Zaragoza (ES)**
• **FRAILE DOLADO, José María**
  **50009 Zaragoza (ES)**
• **GARCÍA MARTÍ, Elena**
  **50009 Zaragoza (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **CONTROLLED RELEASE FORMULATIONS**

(57) In this sense, we herein describe, for the first time, the first report of immobilization of a nonionic drug (dexamethasone) on a Laponite clay, wherein the clay is not used as a component of a polyurethane composite.

EP 3 851 097 A1

**Description**

**Technical field of the invention**

Laponite compositions and methods of using such compositions, useful for injection into the vitreous of human eyes are provided. Such compositions can include active ingredients present in a therapeutically effective amount, and an aqueous carrier component.

**Background of the invention**

Intravitreal injections are invasive and the associated risks (retinal detachments, opportunistic infections, cataracts) increase when repeated applications are required. The short half-life of substances (drugs) administered via this route requires frequent injections for the maintenance of effective concentrations, in the treatment of chronic eye diseases, with poor patient compliance. For this reason, episcleral drug delivery (which includes transcleral and suprachoroidal routes) has become relevant for treatment of posterior segment eye diseases, as a safer route. In base of the short half-life of the drugs, alternative drug delivery systems and sustained-release inserts are being developed to overcome this limitation by reducing the frequency of injections. For example, dexamethasone has been released from polymeric biodegradable implants, in which, the degradation of the polymeric matrix produces a slow and sustained release of dexamethasone. However, one limitation of these inserts is that many of them require a surgical intervention to its implantation.
Therefore, administrating drugs to the posterior segment of the eye is a challenge for the treatment of retinal and choroidal diseases. Such challenge has been targeted by using natural clays, however, Natural clays show a certain degree of variability in their properties, such as chemical composition, particle size, and color. Hence, it is very difficult to envisage their use in intravitreal applications, and only in one case, they have been used as components of polyurethane composites for implants releasing dexamethasone acetate upon degradation of the polymer (Da Silva GR, Ayres E, Orefice RL, Moura SA, Cara DC, Cunha Ada S Jr 2009. Controlled release of dexamethasone acetate from biodegradable and biocompatible polyurethane and polyurethane nanocomposite. J Drug Target 17:374-383).
Therefore, there is still a need to administering drugs, in a controlled released manner, to the posterior segment of the eye for the treatment of retinal and choroidal diseases.

**Brief description of the figures**

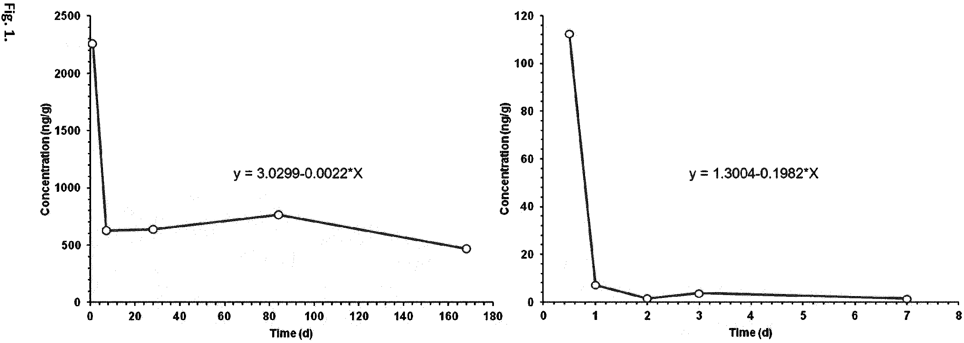

**Fig. 1:** Dexamethasone concentrations in vitreous humor after 100 $\mu$L intravitreal injection of DEX-loaded Laponite (A) and DEX solution (B).

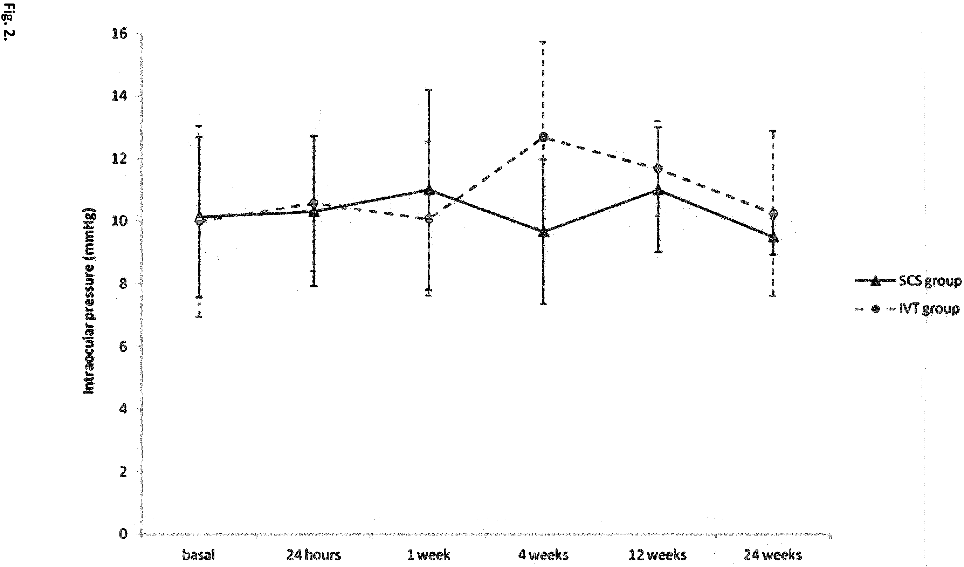

**Fig. 2:** Intraocular pressures changes over time following suprachoroidal (SCS) and intravitreal (IVT) injection of DEX/LAP suspension. Each error bar is constructed using $\pm 1$ standard deviation from the mean.

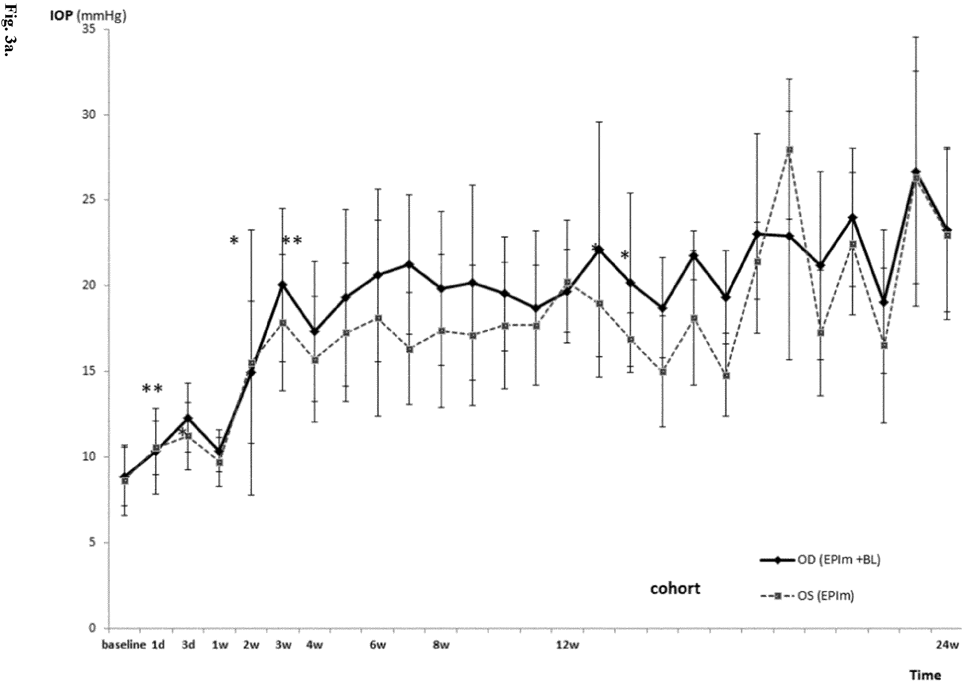

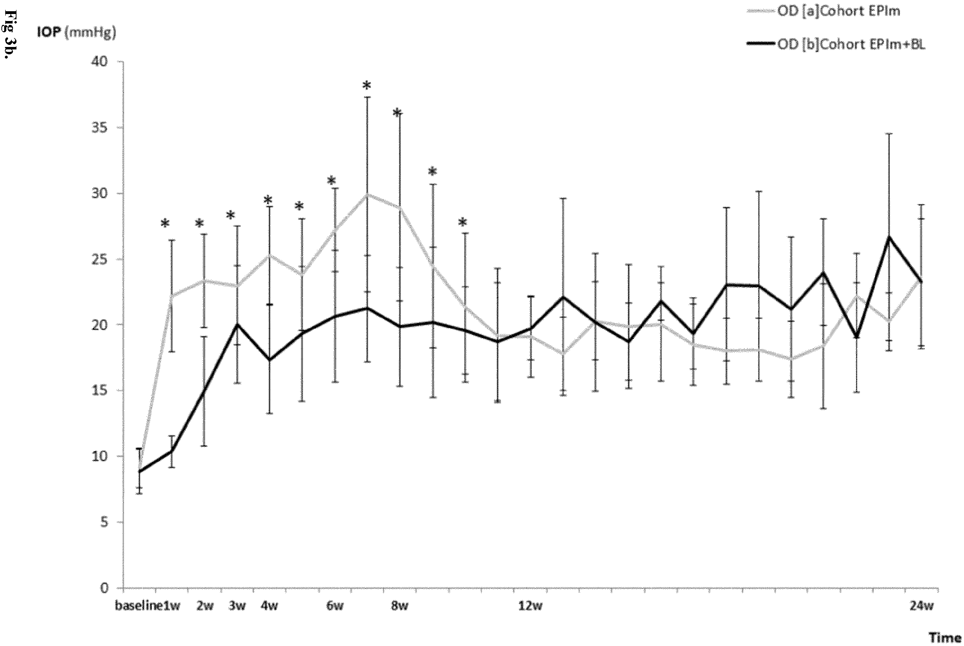

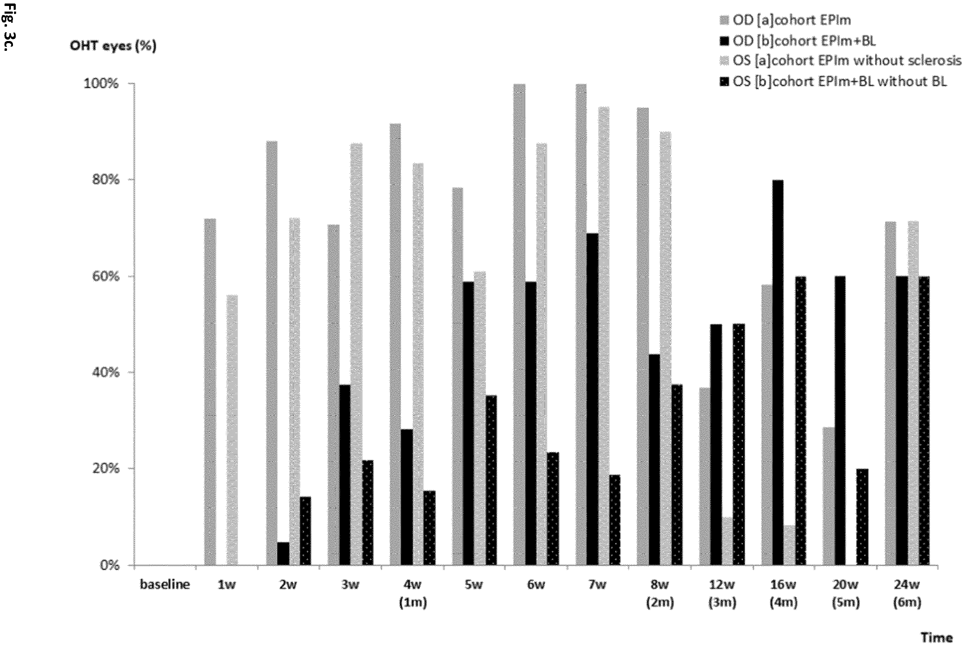

**Fig. 3.** 3a: Curves of intraocular pressure. Comparison between right and left eyes in the [BRI-LAP] cohort (rats with ocular hypertension in both eye and intravitreal injection of Brimonidine-Laponite formulation in right eye). Abbreviations: IOP: intraocular pressure; RE: right eye; LE: left eye; w: week; d: day; *p<0.05: statistical differences, **p<0.001: statistical differences with Bonferroni's correction. RE from [non-bri] cohort received ocular hypertensive injection by sclerosing the episcleral veins, with its contralateral left eye non-intervened. RE from [BRI-LAP] cohort received ocular hypertensive injection by sclerosing the episcleral veins plus intravitreal injection with Brimonidine-Laponite formulation. LE from [BRI-LAP] cohort received ocular hypertensive injection by sclerosing the episcleral veins. Fig 3b: Curves of intraocular pressure. Right eye comparison between [non-bri] cohort (rats with ocular hypertension in right eye) and [BRI-LAP] cohort (rats with ocular hypertension in both eye and intravitreal injection of Brimonidine-Laponite formulation in right eye). Abbreviations: IOP: intraocular pressure; RE: right eye; LE: left eye; w: week; d: day; *p<0.05: statistical differences, **p<0.001: statistical differences with Bonferroni's correction. RE from [non-bri] cohort received ocular hypertensive injection by sclerosing the episcleral veins, with its contralateral left eye non-intervened. RE from [BRI-LAP] cohort received ocular hypertensive injection by sclerosing the episcleral veins plus intravitreal injection with Brimonidine-Laponite formulation. LE from [BRI-LAP] cohort received ocular hypertensive injection by sclerosing the episcleral veins. Fig. 3c: Percentage of ocular hypertensive eyes (right and left eyes) between [non-bri] cohort (rats with ocular hypertension in right eye) and [BRI-LAP] cohort (rats with ocular hypertension in both eye and intravitreal injection of Brimonidine-Laponite formulation in right eye) during the follow-up. Abbreviations: RE: right eye; LE: left eye; w: week; m: month; RE from [non-bri] cohort received ocular hypertensive injection by sclerosing the episcleral veins. LE from [non-bri] cohort did not received any intervention. RE from [BRI-LAP] cohort received ocular hypertensive injection by sclerosing the episcleral veins plus intravitreal

injection with Brimonidine-Laponite formulation. LE from [BRI-LAP] cohort received ocular hypertensive injection by sclerosing the episcleral veins

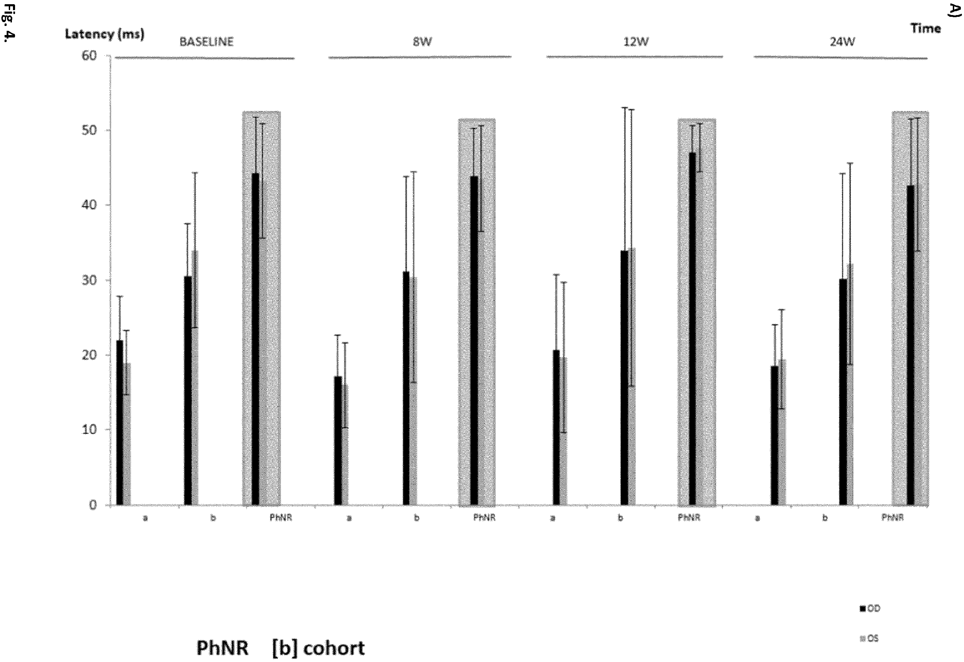

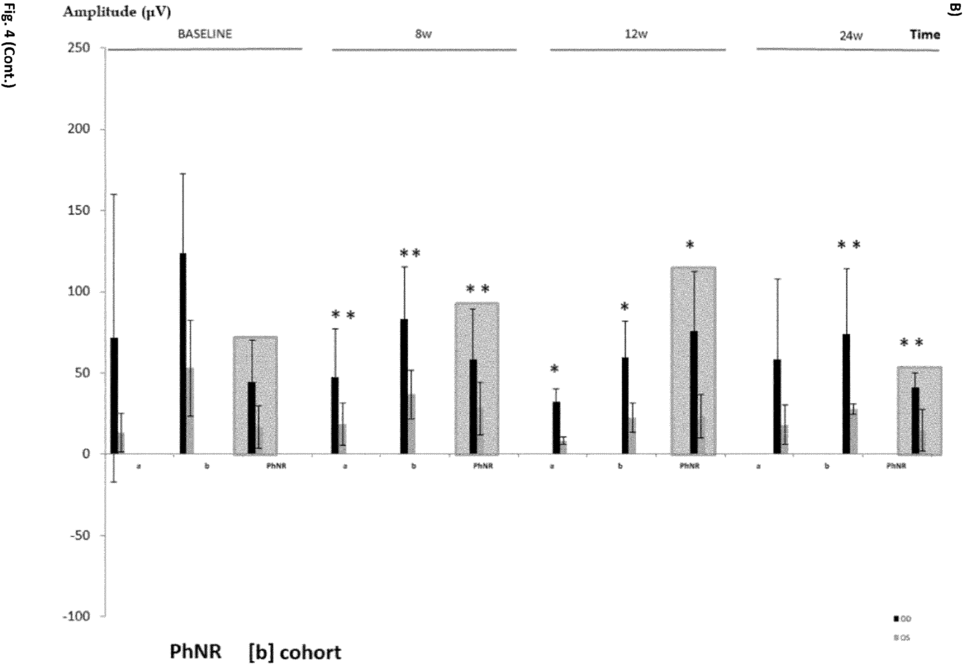

**Fig. 4**: Functional examinations by electroretinography (ERG) **A**: PhNR (Photopic Negative Response) latency from the [BRI-LAP] cohort (rats with ocular hypertension in both eye and intravitreal injection of Brimonidine-Laponite formulation in right eye), maintained over 24 weeks, **B**: PhNR amplitude, (a, b and PhNR waves) increased and was statistically significant higher in eyes treated with the Bri-Lap formulation in comparison with its contralateral left eye,

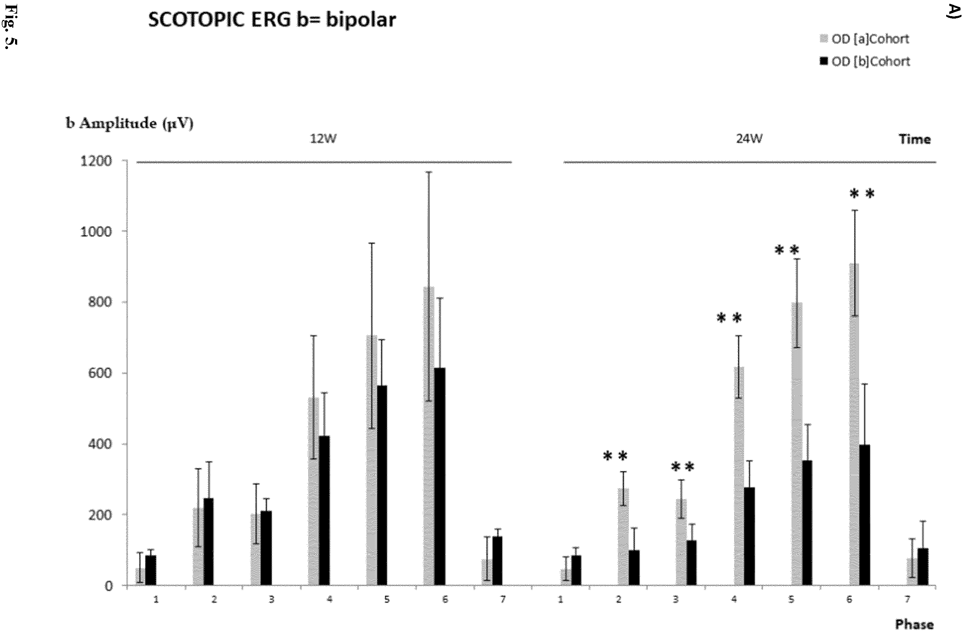

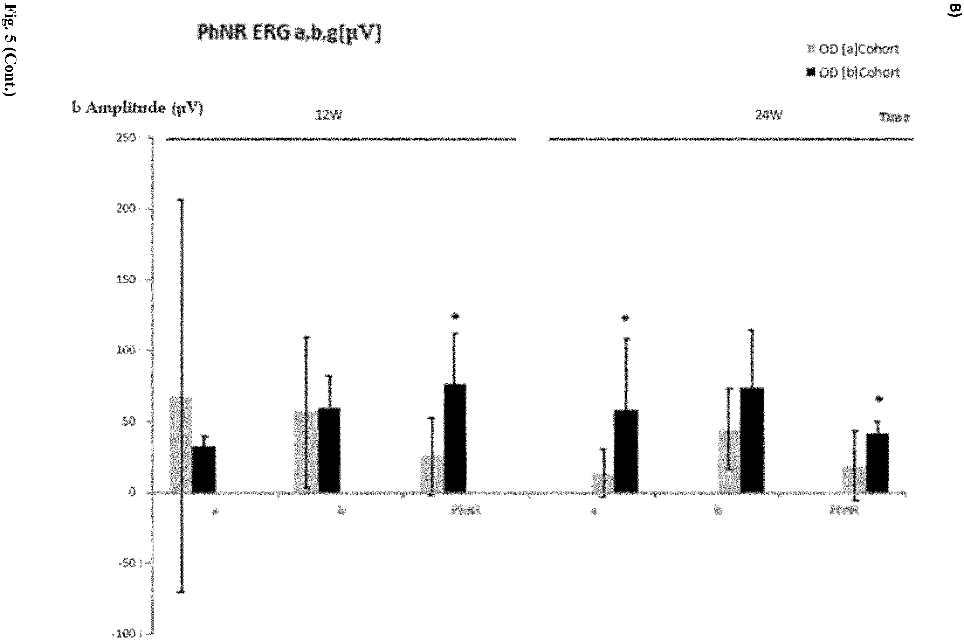

**Fig. 5**: Functional examinations by electroretinography (ERG). **A**: scotopic ERG test showed lower (b wave) amplitude at 24 weeks in eyes treated with the Bri-Lap formulation, **B**: PhNR amplitude, (a and PhNR waves) was statistically significant higher in eyes treated with the Bri-Lap formulation in comparison with hypertensive and non-treated eyes from the [non-bri] cohort. Abbreviations: RE: right eye; LE: left eye; a wave: signal from photoreceptors; b wave: signal from intermediate cells; PhNR wave: signal from retinal ganglion cell; Phases 1 to 7: multistep procedure with increasing intensity of luminance and different intervals from 0.0003 cds/m2 up to 3.0 cds/m2; w: week; ms: milliseconds; $\mu$V: microvolts; *p<0.05: statistical differences, **p<0.001: statistical differences with Bonferroni's correction.

## Description of the invention

### Definitions

As used herein and in the appended claims, the singular forms "a," "an" and "the" include plural referents unless the context clearly dictates otherwise. Thus, for example, reference to "a pore" includes a plurality of such pores and reference to "the drug" includes reference to one or more drugs known to those skilled in the art, and so forth.

Also, the use of "or" means "and/or" unless stated otherwise. Similarly, "comprise," "comprises," "comprising" "include," "includes," and "including" are interchangeable and not intended to be limiting.

It is to be further understood that where descriptions of various embodiments use the term "comprising, " those skilled in the art would understand that in some specific instances, an embodiment can be alternatively described using language "consisting essentially of" or "consisting of."

Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein.

The publications discussed above and throughout the text are provided solely for their disclosure prior to the filing date of the present application. Nothing herein is to be construed as an admission that the inventors are not entitled to antedate such disclosure by virtue of prior disclosure.

As used herein, the words or terms set forth below have the following definitions.

"About" means that the item, parameter or term so qualified encompasses a range of plus or minus ten percent above and below the value of the stated item, parameter or term. "Administration", or "to administer" means the step of giving (i.e. administering) a pharmaceutical composition to a subject. The pharmaceutical compositions disclosed herein can be "locally administered", that is administered at or in the vicinity of the site at which a therapeutic result or outcome is desired. For example to treat an ocular condition (such as for example a macular edema, uveitis or macular degeneration) intravitreal injection or implantation of the composition of the present invention can be carried out. "Sustained release" means release of an active agent (such as a dexamethasone) over a period of about seven days or more, while "extended release" means release of an active agent over a period of time of less than about seven days.

"Entirely free (i.e. "consisting of" terminology) means that within the detection range of the instrument or process being used, the substance cannot be detected or its presence cannot be confirmed.

"Essentially free" (or "consisting essentially of") means that only trace amounts of the substance can be detected.

"Pharmaceutical composition" means a formulation in which an active ingredient (the active agent) can be a steroid, such as a corticosteroid. The word "formulation" means that there is at least one additional ingredient in the pharmaceutical composition besides the active ingredient. A pharmaceutical composition is therefore a formulation which is suitable for diagnostic or therapeutic administration (i.e. by intraocular injection or by insertion of a depot or implant) to a subject, such as a human patient.

"Substantially free" means present at a level of less than one percent by weight of the pharmaceutical composition.

### Description

Synthetic clays, as Laponite (LAP) can be prepared with reproducible composition and textural properties with the additional advantage that LAP forms transparent colloidal dispersions in water making it ideal to intravitreal administration without interfering with vision. Laponite clay refers generally to a synthetic, layered silicate clay that has a layer structure in the form of disc-shaped crystals when dispersed in water. Macromolecules of the laponite clay have a disc-shaped crystal similar to bentonite and hectorite, but are more than one order of magnitude smaller in size. In contrast to clays with a large (e.g., greater than 100) aspect ratio (ratio of length to height) that tend to aggregate in a face-to-face lamellar fashion, laponite clay tends to form partially delaminated disordered aggregates through edge-to-edge interactions. Laponite clay may have, without limitation, an aspect ratio of between about 10 and about 80, or between about 15 and about 50, or between about 20 and about 40, or between about 25 and about 35. In one embodiment, the laponite clay has an aspect ratio of about 30. It will be understood that these aspect ratios represent averages. The surface of the crystal may have a negative charge of about 50-55 mmol·100 g-1. The edges of the crystal may have small, localized positive charges that are about 4-5 mmol 100 g-1. Laponite clay may have a physical surface area over about 900 m2·g-1. Laponite clay may comprise about 8% by weight water that may be released from its crystal structure at temperatures above about 150° C. Commonly known laponite clays encompassed by the invention include without limitation those that comprise: lithium, magnesium, and sodium silicate; those that comprise lithium, magnesium, sodium silicate, and tetrasodium pyrophosphate; those that comprise sodium, magnesium, and those that comprise fluorosilicate; and sodium, magnesium, fluorosilicate, and tetrasodium pyrophosphate. Any combination of two or more of these laponite clays may also be used in the compositions of the invention. Preferably, in the present invention the laponite clay comprises lithium, magnesium, and sodium silicate; preferably +/- 30% of a composition comprising ((w/w%) in percentage by weight over the total weight of the laponite clay composition) silicate (preferably as $SiO_2$) 59.5%, magnesium (preferably as MgO) 27.5%, lithium (preferably as Li2O) 0.8%, sodium (preferably as Na2O) 2.8%, and water ($H_2O$) 9.4%). More preferably +/- 20% of a composition comprising (w/w%) silicate (preferably as $SiO_2$) 59.5%, magnesium (preferably as MgO) 27.5%, lithium (preferably as Li2O) 0.8%, sodium (preferably as Na2O) 2.8%, and water ($H_2O$) 9.4%). More preferably +/- 10% of a composition comprising (w/w%) silicate (preferably as $SiO_2$) 59.5%, magnesium (preferably as MgO) 27.5%, lithium (preferably as Li2O) 0.8%, sodium (preferably as Na2O) 2.8%, and water ($H_2O$) 9.4%). More preferably +/- 1% of a composition comprising (w/w%) silicate (preferably as $SiO_2$) 59.5%, magnesium (preferably as MgO) 27.5%, lithium (preferably as Li2O) 0.8%, sodium (preferably as Na2O) 2.8%, and water ($H_2O$) 9.4%). Still more preferably, a composition consisting of (w/w%) silicate (preferably as $SiO_2$) 59.5%, magnesium (preferably as MgO) 27.5%, lithium (preferably as Li2O) 0.8%, and sodium (preferably as Na2O) 2.8%, and water ($H_2O$) 9.4%). In one embodiment, the diameter of the disc-shaped crystal in the laponite clay may be from about 1 nm to about 300 nm, from about 5 nm to about 150 nm, or from about 10 nm to about 100 nm. In one embodiment, the diameter of the disc-shaped crystal is from about 15 nm to about 50 nm. In another embodiment, the diameter of the disc-shaped crystal is from about 20 nm to about 40 nm, or from about 25 nm to about 35 nm, or about 25 nm, or about 35 nm.

In one embodiment, the thickness of the disc-shaped crystal in the laponite clay may be from about 0.1 nm to about 3 nm, from about 0.5 nm to about 1.5 nm, or from about 0.8 nm to about 1.2 nm. In one embodiment, the thickness of the disc-shaped crystal is from about 0.8 nm to about 1 nm. In another embodiment, the thickness of the disc-shaped crystal is about 0.92 nm. In one embodiment, the density of laponite clay is between about 2.5 and about 2.55 gm/cm3, or about 2.53 gm/cm3.

Non-limiting examples of laponite are disclosed in Herrera et al., J. Mater. Chem., 2005, 863-871; and Cummins, J. Non-Crystalline Solids, 2007, 353, 3891-3905, the disclosures of which are hereby incorporated by reference. Suitable laponites are available from BYK as either gel-forming or sol-forming grades. Gels and sols comprising laponites may be thixotropic and/or shear thinning Special mention may be made of the sol forming grade S482.

In this sense, we herein describe, for the first time, the first report of immobilization of a non-ionic drug (dexamethasone) on a Laponite clay, wherein the clay is not used as a component of a polyurethane composite. As shown in example 1, the nature of the drug-clay interaction and the *in vitro* behavior of the material in controlled delivery, demonstrates that LAP succeeded in delivering dexamethasone at an almost constant rate for 24 weeks. In addition, the intravitreal injections of 100 μL dexamethasone/laponite dispersion (10 mg/mL, 1:10 w/w) were performed in albino rabbits and DXM concentrations in vitreous humor were monitored over 24 weeks. Vitreous DXM levels remained relatively stable from 7th day post-administration and showed an elimination rate significantly lower than that found after intravitreal injection of dexamethasone in solution (1 mg/mL) ($C_L$ = 0.49 vs 315.29 g/day, respectively). The administration of DXM loading to laponite can lengthen the half-life of the drug ($T_{1/2-el}$ = 132.75 days vs 0.13 days) and enhance its clinical uses. The single intravitreal injection of DEX/LAP provided a sustained release of the corticosteroid for up to 17 weeks.

On the other hand, suprachoroidal administration is considered a promising alternative for treatment of posterior segment eye diseases, however, its applicability has been limited by the need for a reliable and minimally invasive way to access the suprachoroidal space. The injection technique varies between the search groups. We have used the technique who accessed the SCS using surgical procedures which require a scleral incision and advancement of a long cannula or hypodermic needle. Other authors assess the administration to the SCS using hollow microneedles allowing minimally invasive injection which could be accomplished within few seconds. Drug formulation could be a major factor in optimizing pharmacokinetic and pharmacodynamics using suprachoroidal administration. One of the limitations of the present study was that DEX levels were computed from a retinal-choroidal compound. This could lead to a cross-contamination between the retina and the choroid during the dissection procedure.

The fellow eyes were not analyzed for the SCS group because the drug source for these eyes would be from blood circulation and DEX in blood would be very low for this route.

In contrast with the acute elevations of IOP observed after suprachoroidal injection, with larger injection volumes (> 100-150 μL), in the current study there was a drop of intraocular pressure in the 4th week postadministration. Equivalent volume of LAP solution (without DEX loaded) led to a smaller IOP elevation.

Signs of ocular surface inflammation observed during the first week after the suprachoroidal administration should be attributable to the surgical procedure performed, and it has been described in those cases where cannulation of the suprachoroidal space was used versus injections into the SCS using microneedles.

Laponite was thus able to increase the rate of dexamethasone release when compared to the rate of drug release from conventional solutions. Laponite provided a sustained release of dexamethasone and lengthen its time of permanence in vitreous humor when compared to conventional solutions and enhance its clinical use for the treatment of chronic ocular diseases. In addition, and as shown in example 2, the results further demonstrate that Brimonidine-Laponite (Bri-Lap) formulations had a protective effect over the structure of Retina at early times (weeks 0 to 6); as well as axonal and ganglionar at intermediate and late times (weeks 6 to 24) if it was intravitreally injected in an eye with OHT (Ocular hypertension). Moreover, Bri-Lap intravitreal formulations not only exerted axonal and ganglionar protection in the injected eye from week 6, but also in its contralateral non-injected eye at week 24, and thickness measurements were even higher than those from eyes non inducted.

Therefore, a first aspect of the invention refers to, a composition (from hereinafter "LAP formulation") for the sustained delivery of non-ionic drugs, preferably non-cationic drugs, wherein the composition comprises laponite, or any other synthetic silicate-based solid with clay-like laminar structure capable of forming gels in water, and a non-cationic drug, and wherein the laponite acts as a carrier for the controlled delivery of the non-ionic drug, with the proviso that said composition does not comprise polyurethane. Preferably, the composition is in the form of a powder. Preferably, the composition is a homogenous, preferably transparent, dispersion or suspension suitable to be injected intravitreously or suprachoroidally. Preferably, the ratio of the non-ionic drug and the synthetic silicate-based solid with clay-like laminar structure present in the LAP formulation is from 1:3 to 1:25, preferably between 1:5 and 1:15. Preferably, the non-ionic drug is selected from the group consisting of dexamethasone, or Brimonidine (br).

In the context of the present invention other synthetic silicate-based solids with clay-like laminar structure capable of forming gels in water can be selected from any aluminium-silicate, magnesium-silicate, mixed oxides and hydroxides, with inorganic or organic compensating cations or anions capable of forming transparent colloidal dispersions in water for intravitreal administration without interfering with vision.

The present invention is thus based upon our discovery of LAP formulations specifically designed for intravitreal injection to treat various ocular conditions, such a macula edema. Our LAP formulations have numerous superior characteristics and advantages; in particular these formulations can provide sustained release of therapeutic amounts of the non-ionic, preferably non-cationic, drug over multi-month periods upon intravitreal injection of such formulations; in addition, intravitreal administration of our LAP formulations is not associated with an increased incidence of adverse events such as elevated intra ocular pressure, glaucoma, cataract and/an intraocular inflammation; moreover, intravitreal administration of our LAP formulations is not only not associated with an increased incidence of adverse events such elevated intra ocular pressure, glaucoma, cataract and/an intraocular inflammation, but to the contrary, these formulations are especially useful in the treatment of ocular hypertension; lastly, the sustained release characteristic of our formulations reduces the need for intravitreal administration of large drug quantities to achieve a desired therapeutic effect; upon intravitreal administration.

Generally, the present invention provides compositions useful for placement, preferably by injection, into a posterior segment of an eye of a human or animal. Such compositions in the posterior, e.g., vitreous, of the eye are therapeutically effective against one or more conditions and/or diseases of the posterior of the eye, and/or one or more symptoms of such conditions and/or diseases of the posterior of the eye.

It is important to note that while the compositions disclosed herein are preferably administered by intravitreal injection to treat a posterior ocular condition, our compositions can also be administered (as by injection) by other routes, such as for example subconjunctival, sub-tenon, periocular, retrobulbar, suprachoroidal, and/or intrascleral to effectively treat an ocular condition. Additionally, a sutured on refillable dome can be placed over the administration site to prevent or to reduce wash out, leaching and/or diffusion of the active agent in a non-preferred direction.

Compositions within the scope of our invention can comprise any non-ionic drug, preferably non-cationic, drug such as a corticosteroid component as dexamethasone or brimonidine, and an aqueous carrier component. The compositions are advantageously ophthalmically acceptable. One of the important advantages of the present compositions is that they are compatible with or friendly to the tissues in the posterior segment of the eye, for example, the retina of the eye. As noted above, the present compositions may include a corticosteroid component. Such corticosteroid component is present in the compositions in a therapeutically effective amount, that is in an amount effective in providing a desired therapeutic effect in the eye into which the composition is placed. Any suitable non-ionic, preferably non-cationic, corticosteroid component may be employed in according to the present invention. Examples of useful non-ionic corticosteroid components include, without limitation, cortisone, prednisolone, triamcinolone, triamcinolone acetonide, fluorometholone, dexamethasone, medrysone, loteprednol, derivatives thereof and mixtures thereof. Further examples of useful drug components include selective alpha-adrenergic receptor agonists such as Brimonidine or derivatives thereof. As used herein, the term "derivative" refers to any substance which is sufficiently structurally similar to the material of which it is identified as a derivative so as to have substantially similar functionality or activity, for example, therapeutic effectiveness, as the material when the substance is used in place of the material. In one very useful embodiment, the corticosteroid component comprises dexamethasone.

The corticosteroid component advantageously is present in an amount of at least about 10 mg per ml of the composition. One important advantage of the present invention is the effective ability of the present compositions to include relatively large amounts or concentrations of the corticosteroid component. Thus, the corticosteroid component may be present in the present compositions in an amount in the range of about 1 % or less to about 5% or about 10% or about 20% or about 30% or more (w/v) of the composition, as long as the ratios identified above are maintained. Providing relatively high concentrations or amounts of corticosteroid component in the present compositions is beneficial in that reduced amounts (volumes for injection) of the composition may be required to be placed or injected into the posterior segment of the eye. Thus, in one very useful embodiment, the present compositions include more than about 4% (w/v), for example at least about 5% (w/v), to about 10% (w/v) or about 20% (w/v) or about 30% (w/v) of the corticosteroid component.

The aqueous carrier component is advantageously ophthalmically acceptable and may include one or more conventional excipients useful in ophthalmic compositions. The present compositions preferably include a major amount of liquid water. The present compositions may be, and are preferably, sterile, for example, prior to being used in the eye. The present compositions preferably include at least one buffer component in an amount effective to control the pH of the composition and/or at least one tonicity component in an amount effective to control the tonicity or osmolality of the compositions. More preferably, the present compositions include both a buffer component and a tonicity component. The buffer component and tonicity component may be chosen from those which are conventional and well known in the ophthalmic art. Examples of such buffer components include, but are not limited to, acetate buffers, citrate buffers, phosphate buffers, borate buffers and the like and mixtures thereof. Phosphate buffers are particularly useful. Useful tonicity components include, but are not limited to, salts, particularly sodium chloride, potassium chloride, mannitol and other sugar alcohols, and other suitable ophthalmically acceptably tonicity component and mixtures thereof. The amount of buffer component employed preferably is sufficient to maintain the pH of the composition in a range of about 6 to about 8, more preferably about 7 to about 7.5. The amount of tonicity component employed preferably is sufficient to provide an osmolality to the present compositions in a range of about 200 to about 400, more preferably about 250 to about 350, mOsmol/kg respectively. Advantageously, the present compositions are substantially isotonic.

The present compositions may include one or more other components in amounts effective to provide one or more useful properties and/or benefits to the present compositions. For example, although the present compositions may be substantially free of added preservative components, in other embodiments, the present compositions include effective amounts of preservative components, preferably such components which are more compatible with or friendly to the tissue in the posterior segment of the eye into which the composition is placed than benzyl alcohol. Examples of such preservative components include, without limitation, benzalkonium chloride, chlorhexidine, PHMB (polyhexamethylene biguanide), methyl and ethyl parabens, hexetidine, chlorite components, such as stabilized chlorine dioxide, metal chlorites and the like, other ophthalmically acceptable preservatives and the like and mixtures thereof. The concentration of the preservative component, if any, in the present compositions is a concentration effective to preserve the composition and is often in a range of about 0.00001% to about 0.05% or about 0.1% (w/v) of the composition.

In addition, the present composition may include an effective amount of resuspension component effective to facilitate the suspension or resuspension of the drug component particles in the present compositions. As noted above, in certain embodiments, the present compositions are free of added resuspension components. In other embodiments of the present compositions effective amounts of resuspension components are employed, for example, to provide an added degree of insurance that the corticosteroid component particles remain in suspension, as desired and/or can be relatively easily resuspended in the present compositions, such resuspension be desired. The presently useful resuspension components are present, if at all, in the compositions in accordance with the present invention in an amount effective to facilitate suspending the particles in the present compositions, for example, during manufacture of the compositions or thereafter. The specific amount of resuspension component employed may vary over a wide range depending, for example, on the specific resuspension component being employed, the specific composition in which the resuspension component is being employed and the like factors. Suitable concentrations of the resuspension component, if any, in the present compositions are often in a range of about 0.01 % to about 5%, for example, about 0.02% or about 0.05% to about 1.0% (w/v) of the composition.

The present LAP formulations or compositions can be prepared as indicated in example 1. The preparation processing should be chosen to provide the present compositions in forms which are useful for placement or injection into the posterior segments of eyes of humans or animals.

Methods of using the present composition are provided and are included within the scope of the present invention. In general, such methods comprise administering a composition or LAP formulation in accordance with the present invention to a posterior segment of an eye of a human or animal, thereby obtaining a desired therapeutic effect. The administering step advantageously comprises at least one of intravitreal injecting, subconjunctival injecting, sub-tenon injecting, retrobulbar injecting, suprachoroidal injecting and the like. A syringe apparatus including an appropriately sized needle, for example, a 27 gauge needle or a 30 gauge needle, can be effectively used to inject the composition with the posterior segment of an eye of a human or animal. Ocular conditions which can be treated or addressed in accordance with the present invention include, without limitation, the following: Maculopathies/retinal degeneration: macular degeneration, including age related macular degeneration (ARMD), such as non-exudative age related macular degeneration and exudative age related macular degeneration, choroidal neovascularization, retinopathy, including diabetic retinopathy, acute and chronic macular neuroretinopathy, central serous chorioretinopathy, and macular edema, including cystoid macular edema, and diabetic macular edema. Uveitis/retinitis/choroiditis: acute multifocal placoid pigment epitheliopathy, Behcet's disease, birdshot retinochoroidopathy, infectious (syphilis, lyme, tuberculosis, toxoplasmosis), uveitis, including intermediate uveitis (pars planitis) and anterior uveitis, multifocal choroiditis, multiple evanescent white dot syndrome (MEWDS), ocular sarcoidosis, posterior scleritis, serpignous choroiditis, subretinal fibrosis, uveitis syndrome, and Vogt-Koyanagi-Harada syndrome. Vascular diseases/exudative diseases: retinal arterial occlusive disease, central retinal vein occlusion, disseminated intravascular coagulopathy, branch retinal vein occlusion, hypertensive fundus changes, ocular ischemic syndrome, retinal arterial microaneurysms, Coat's disease, parafoveal telangiectasis, hemi-retinal vein occlusion, papillophlebitis, central retinal artery occlusion, branch retinal artery occlusion, carotid artery disease (CAD), frosted branch angitis, sickle cell retinopathy and other hemoglobinopathies, angioid streaks, familial exudative vitreoretinopathy, Eales disease. Traumatic/surgical: sympathetic ophthalmia, uveitic retinal disease, retinal detachment, trauma, laser, PDT, photocoagulation, hypoperfusion during surgery, radiation retinopathy, bone marrow transplant retinopathy. Proliferative disorders: proliferative vitreal retinopathy and epiretinal membranes, proliferative diabetic retinopathy. Infectious disorders: ocular histoplasmosis, ocular toxocariasis, presumed ocular histoplasmosis syndrome (POHS), endophthalmitis, toxoplasmosis, retinal diseases associated with HIV infection, choroidal disease associated with HIV infection, uveitic disease associated with HIV Infection, viral retinitis, acute retinal necrosis, progressive outer retinal necrosis, fungal retinal diseases, ocular syphilis, ocular tuberculosis, diffuse unilateral subacute neuroretinitis, and myiasis. Genetic disorders: retinitis pigmentosa, systemic disorders with associated retinal dystrophies, congenital stationary night blindness, cone dystrophies, Stargardt's disease and fundus flavimaculatus, Bests disease, pattern dystrophy of the retinal pigmented epithelium, X-linked retinoschisis, Sorsby's fundus dystrophy, benign concentric maculopathy, Bietti's crystalline dystrophy, pseudoxanthoma elasticum. Retinal tears/holes: retinal detachment, macular hole, giant retinal tear. Tumors: retinal disease associated with tumors, congenital hypertrophy of the RPE, posterior uveal melanoma, choroidal hemangioma, choroidal osteoma, choroidal metastasis, combined hamartoma of the retina and retinal pigmented epithelium, retinoblastoma, vasoproliferative tumors of the ocular fundus, retinal astrocytoma, intraocular lymphoid tumors. Miscellaneous: punctate inner choroidopathy, acute posterior multifocal placoid pigment epitheliopathy, myopic retinal degeneration, acute retinal pigment epitelitis and the like. The present methods may comprise a single injection into the posterior segment of an eye or may involve repeated injections, for example over periods of time ranging from about one week or about 1 month or about 3 months to about 6 months or about 1 year or longer.

The following examples serve to illustrate the present invention but do not limit the same.

**Examples**

**Example 1.**

**Material and Methods**

**Chemical and reagents**

Dexamethasone (DEX) was from Sigma-Aldrich (Madrid, Spain). Laponite®-RD (LAP) (surface density 370 m2/g, bulk density 1000 kg/m3, chemical composition: $SiO_2$ 59.5%, MgO 27.5%, $Li_2O$ 0.8%, $Na_2O$ 2.8%, $H_2O$ 9.4%) was purchased from BYK Additives Ltd. (Widnes, Cheshire, UK). Sodium hyaluronate 3% solution (Healon® EndoCoat OVD) and balanced 0.9% salt solution (9 mg/mL NaCl) (BSS) were obtained from Abbott Medical Optics (Barcelona, Spain) and Fresenius Kabi (Barcelona, Spain), respectively. Ethanol, acetone and acetonitrile were HPLC grade from Scharlab (Barcelona, Spain).

**Preparation and characterization of formulation DEX/LAP**

The optimal concentration of dexamethasone (DEX) to load to Laponite has been determined on the basis of the nature of the drug-clay interaction and the in vitro behaviour of the medium to the release of the drug. The drug was incorporated into the clay by addition of LAP into a solution of drug in an organic solvent: 10 mg-DEX were dissolved in acetone (10 mL) and then 100 mg-LAP were added to this solution. The slurry was stirred at room temperature for 1 hour and then the solvent was removed in a rotary evaporator. The solid was dried at room temperature overnight under vacuum over $P_2O_5$. DEX/LAP powder was stored in tightly capped single-use vials that were gamma ray sterilized.

Immediately before injection, DEX/LAP powder was suspended in 10 mL of BSS and gently vortexed for 10 minutes, to yield a transparent and homogeneous dispersion (10 mg/mL; 1:10 w/w). A 100 $\mu$L and 50 $\mu$L-volume of DEX/LAP suspension was then injected intravitreously or suprachoroidally, respectively.

**Study design**

All experiments were carried out using female New Zealand Albino rabbits. Animal handling was in accordance to the Spanish Policy for Animal Protection RD1201/05, which meets the European Union Directive 86/609/EEC. Practices and animal care complied with the ARVO Statement for the Use of Animals in Experimental Procedures and Other Scientific Purposes. All procedures were performed according to the Project License PI12/02285 approved by the in-house Ethics Committee for Animal Experiments of the University of Zaragoza (Spain). The animals were singly housed in metabolic standard cages, in a light-controlled room (12 h/12 h dark/light cycle) at $20\pm2°$ C with a relative humidity of 40-70%. Diet and water were available ad libitum and thorough clinical examination was performed daily.

**In vivo administration: injection procedure**

Thirty animals weighing 2.5 - 4.0 kg (3.1 $\pm$ 0.4 kg) were included in the study and randomly divided into two groups to test suprachoroidal (SCS group) or intravitreal (IVT group) administration of DEX/LAP formulation.

All the injections were performed under general anaesthesia and aseptic conditions. Animals were anaesthetized by intramuscularly injecting ketamine hydrochloride (25 mg/Kg) (Ketolar 50® Pfizer, Madrid, Spain) and medetomidine (0.5 mg/Kg) (Domtor®, Esteve, Madrid, Spain). Topical anaesthesia with tetracaine clorhydrate (1 mg/mL) and oxibuprocaine clorhydrate (4 mg/mL) ophthalmic drops (Colircusí Anestésico Doble®, Alcon Cusí SA, Barcelona, Spain) was used, and povidone-iodine solution (5%) was applied for ocular surface antisepsis before and after the injection.

The same ophthalmologist performed all the injections, under the direct view of a surgical microscope (Zeiss Opmi 6c/Osmi 99 Microscope, Carl Zeiss Meditec Inc, California, USA). An adequate exposure of the ocular globe was obtained by placing an eyelid speculum (Moria SA, Antony, France).

Only one eye of each animal was administered (right eye). The fellow eye (left eye) remained untreated and served as the comparative control.

*Determination of injection volumes*

The dose of dexamethasone used was 0.05 mg in 50 $\mu$L for suprachoroidal injection and 0.1 mg in 100 $\mu$L for intravitreal injection, which is the optimal concentration of dexamethasone (DEX) to load to laponite clay (10 mg/mL, 1:10 w/w). The volume of suspension used for suprachoroidal administration was determined as the volume that could be safely administered without risk of reflux or inducting intraocular pressure elevation.

*Suprachoroidal injection*

We first tested the procedure to access the suprachoroidal space and determined the extent to which a 50 $\mu$L-LAP suspension remains into the suprachoroidal space up to 14 weeks postadministration (about 13% of total initial administered dose).

The suprachoroidal administration was performed in 15 animals. In this group, general inhalation anaesthesia with 2.5% sevoflurane (Sevorane®, Abbott Laboratories, Madrid, Spain) in oxygen via mask, and control of vital signs were performed during the procedure.

The administration was carried out in the superior nasal quadrant of the eye, by performing a conjunctival peritomy with a radial cut parallel to the superior rectus muscle, to expose the sclera. A deep intrascleral incision was made about 9 mm past the limbus, near the globe meridian of 2 o'clock. The suprachoroidal space was entered using a blunt spatula for sharp dissection across the sclera, making a tiny pocket. Direct administration of 50 $\mu$L DEX/LAP-suspension (10 mg/mL; 1:10 w/w) into the pocket was performed by injection with a 25-gauge irrigating cannula (angled 35°, 7.0 mm from bend to tip, 19.0 mm overall length excluding hub) attached to a 1 mL-syringe and compressing the port of the pocket with two surgical microsponges to prevent reflux before removing the cannula. After the procedure, the conjunctiva was restored by an 8-0 suture and povidone-iodine solution (5%) eye drops were applied.

Following suprachoroidal administration the animals were allowed to recover from the anaesthesia, and the health state of the eye surface was monitored.

*Intravitreal injection*

The other fifteen animals received an intravitreal injection of 100 μL-DEX/LAP suspension (10 mg/mL; 1:10 w/w) with a 25-gauge needle in the superior temporal quadrant, about 3-5 mm posterior to the limbus, towards the center of the vitreous cavity. Previously, a paracentesis in the anterior chamber was performed using a 30-gauge hypodermic needle with removal of aqueous humor, to avoid an intraocular pressure elevation. After the injection, the absence of reflux was verified in all cases by compression of the injection point with a cotton swab for 30 seconds.

After administration, the eyes were monitored by ocular tonometry, slit lamp examination and indirect ophthalmoscopy, before sacrifice as previously reported.

**Pharmacokinetic study**

Thirty female NZ albino rabbits were used for this study: fifteen for SCS group (50 μL-DEX/LAP suspension) and other fifteen for IVT group (100 μL).

After administration of DEX/LAP suspension (10 mg/mL, 1:10 w/w), the rabbits were sacrificed for sampling eye tissues and subsequent ocular PK studies. Three animals were used for each sampling time point at 1, 7, 28, 84 and 168 days. A rapid intravenous injection of sodium pentobarbital (30 mg/kg) through the ear vein was performed and immediately after euthanasia, ocular tissues were collected. Following eye enucleation, the globes were snap-frozen (-40°C) and specimens were obtained by eye dissection (vitreous, lens, sclera and chorio-retina compound). DEX concentrations in ocular tissues were determined using a simple and easily accessible method by HPLC-Mss, according to the methodology described by our group in a previous study.

*Analytical method*

The determination of DEX was carried out by HPLC-Mss method in a Waters 2695 system equipped with a Phenomenex Kinetex C18 column (75 mm × 4.6 mm × 2.6 μm) coupled to a Waters 2995 PDA detector. The mobile phase was acetonitrile/water (35:65 v/v) pumped at a flow rate of 0.2 mL/min at 35°C. Detection at 250 nm was used. The amount of DEX was calculated by a seven point calibration curve against 6-α-methylprednisolone as internal standard. Aliquots of the extraction medium (200 μL) were mixed with acetonitrile (2 mL) and 100 μL of a standard solution (20 ppm in acetonitrile). The solution was then vortexed for 1 min and sonicated for 5 min to ensure the complete mixing, and finally centrifuged at 3000 rpm for 5 min. The supernatant was collected and evaporated under vacuum, and then dissolved in 200 μL of acetonitrile, filtered through a 0.22 μm PTFE syringe filter and analyzed.

*Pharmacokinetic and statistical Analysis*

Dexamethasone concentrations were determined to perform pharmacokinetics analysis (PK) in different tissues respectively. PK data were analyzed for the best fit and were consequently modelled according to compartmental models using Microsoft Excel's "PKSolver Add-in" (Albuquerque, NM, USA), in which equations describing concentration as a function of time are used. The following PK parameters were obtained: peak concentration ($C_{max}$), time to peak concentration ($T_{max}$), elimination half-life ($T_{1/2}$).

To describe the time course-concentration after administration the following equation was used:

$$C_{tissue} = C_0 * e^{-K_e t}$$

where $C$ is the tissue concentration, $C_0$ is the concentration at time to, $K_e$ is the elimination rate constant which is derived from the elimination half-life of the drug ($K_e$ = 0.693/$T_{1/2}$). The drug clearance ($C_L$) was calculated according to the following equation:

$$Cl_{tissue} = \frac{Dose}{AUC}$$

where the *Dose* administered was 0.1 mg of dexamethasone for IVT group and 0.05 mg for SCS group, and $AUC_{0-\infty}$ the area under the concentration-time curve estimated by the linear-trapezoidal method from the experimental tissue concentrations, in which the area from the last concentration point ($T_{last}$= day 168) to infinity ($\infty$) was calculated as $C_{last}/K_e$.

Pharmacokinetics of intravitreal DEX solution was not concurrently performed because such a study was already conducted by our group.

Statistical analyses were performed by SPSS version 21.0 (SPSS Inc., Chicago, IL). Data of dexamethasone concentrations were expressed as mean $\pm$ standard deviation. The Kolmogorov-Smirnov test was used to determine goodness of fit to the normal distribution of the continuous variables. The Student's t-test was used to compare tissue concentrations and PK parameters between the two groups. Other results were descriptive measures. Pearson's Chi-Square test was used to compare categorical data. Statistical significance was accepted at a level of $p< 0.005$.

**Safety study of DEX/LAP suspension administration**

Two rabbits were used for this study: 1 for tested suprachoroidal route and 1 for intravitreal injection. Therefore, two eyes of each animal were administered to reduce animal usage.

**Results**

**Concentration of dexamethasone**

The mean levels of dexamethasone concentrations in the posterior segment tissues (vitreous, chorio- retinal compound, sclera and lens) at different time points following IVT and SCS administration are shown in table 1, and the results of the concentration-time curves are presented in figure 1.

| | Vitreous humor (ng/g) | Lens (ng/g) | Choroido-retinal compound (ng/g) | Sclera (ng/g) |
|---|---|---|---|---|
| **SCS group** | | | | |
| 1 d | 305.07 ± 168.99 | 24750.52 ± 7577.00 | - | 283.53 ± 318.02 |
| 7 d | 460.38 ± 93.79 | 263.34 ± 30.66 | 275.54 ± 164.85 | 229.63 ± 35.54 |
| 28 d | 108.03 ± 187.12 | 122.02 ± 72.30 | 101.66 ± 88.12 | 89.69 ± 77.68 |
| 84 d | - | 24.14 ± 41.82 | 12.28 ± 21.27 | 28.86 ± 49.99 |
| 168 d | - | - | 12.04 ± 20.85 | 25.46 ± 44.09 |
| **IVT group** | | | | |
| 1 d | 2258.00 ± 1610.32 | - | - | - |
| 7 d | 626.42 ± 251.31 | - | - | - |
| 28 d | 636.94 ± 206.43 | - | - | - |
| 84 d | 763.46 ± 267.84 | - | - | - |
| 168 d | 466.32 ± 311.15 | - | - | - |

Table 1: Dexamethasone concentrations in posterior segment eye after suprachoroidal administration of 50 μL (10 mg/mL; 1:10 w/w) DEX/LAP suspension and after intravitreal injection of 100 μL-DEX/LAP suspension.

For suprachoroidal administración, dexamethasone levels could be detected in choroido-retinal compound of the injected eyes until 24 weeks post-administration even though non-quantifiable levels were found in vitreous body at this time. DEX levels in choroido-retina start being detectable at 7th day postadministration. The highest DEX concentrations in choroido-retina (275.54 ± 164.85 ng/g) were observed in the first week, followed by a plateau (12.28 ± 21.27 ng/g) from 12th week until week 24. Levels of DEX in vitreous humor were significantly higher at 1, 7 and 28 days in IVT group, and not detectable 84 days in SCS group. Levels of DEX in the choroido-retinal compound were negligible after intravitreal administration because they were under the lowest limit of quantification (LLOQ: 45 ng/g) for the analytical method. The highest intraocular DEX levels were observed in lens (24750.52 ± 7577.00 ng/g) at the first day post-administration in SCS group.

**Ocular tissue pharmacokinetics**

Time course of the experimental vitreous levels from 100 μL DEX/LAP intravitreal injection could be explained by a non-compartmental model with a good correlation between observed and predicted concentrations ($R^2$= 0.9897). Parmacokinetic parameters of dexamethasone in rabbits' vitreous humor after IVT route are shown in table 2 and compared with those obtained after intravitreal injection of DEX in solution (1 mg/mL).

Table 2: Ocular tissue pharmacokinetic parameters following 100 μL-intravitreal administration of DEX/LAP suspension (10 mg/mL, 1:10 w/w) and DEX solution (1 mg/mL).

| PK parameters | Units | DEX solution (1 mg/mL) | DEX/LAP dispersion (10 mg/mL (1:10 w/w)) |
|---|---|---|---|
| $C_0$ | ng/g | 1737.71 | 2795.98 |
| $K_e$ | $d^{-1}$ | 5.48 | 0.005 |
| $T_{1/2}$ | d | 0.13 | 134.75 |
| $v_{ss}$ | g | 57.55 | 97.57 |
| $C_l$ | g/d | 315.29 | 0.49 |
| $AUC_{0-\infty}$ | ng/g*d | 317.17 | 205968.47 |

Following IVT route, the maximum concentration of dexamethasone (Cmax) was 2795.98 ng/g in vitreous body after DEX/LAP. No levels were detectable in choroido-retinal compound, sclera, or lens, respectively. Intravitreal injection of DEX/LAP dispersion showed an elimination rate significantly lower than that found after intravitreal injection of dexamethasone in solution, with a clearance of 0.49 vs 315.29 g/day, respectively. The administration of DEX loading to laponite can lengthen the half-life of the drug from 0.13 days (≈ 3 hours) to 134.75 days (≈ 4.5 months).

The values of intraocular pressure (IOP) after DEX/LAP-suspension administration ranged from 7 to 16 mmHg and no significant differences were found between the two groups. In suprachoroidal group there was a drop of intraocular pressure in the 4th week postadministration.

**Example 2**

**MATERIAL Y METHODS**

**Animals**

The study was carried out on 92 (40% males, 60% females) Long Evans rats 4-week-old of age and weights ranged from 50-100 grams at the beginning of the study. Animals were housed in standard cages with water and food ad libitum, in rooms under controlled temperature (22°C) and relative humidity (55%) with 12-hours dark-light cycles. The work with animals was carried out in the experimental surgery service department of the Biomedical Research Center of Aragon (CIBA). The experiment was previously approved by the Ethics Committee for Animal Research (PI34/17) and carried out in strict accordance with the Association for Research in Vision and Ophthalmology's Statement for the use of Animals.

**Ocular hypertension (OHT) induction and drug injection procedure.**

Animals were divided in [a]cohort and [b]cohort. [a]cohort was compound by 31 rats. In this cohort OHT was induced in right eye (OD) and left eye (OS) was kept without intervention and served as control eye. [b]cohort was compound by 60 rats. In this cohort OHT was induced in both eyes but OD received an intravitreal injection with Brimonidine-Laponite (Bri-Lap) formulation. OD served as treated hypertensive eye and OS as hypertensive control eye.

Ocular hypertension was generated with the model described by Morrison et al by means of sclerosis of episcleral veins (Morrison JC et al 1997) with a hypertonic 1.8M solution under topical eye drops (Anestesico doble Colircusi®, Alcon Cusí® SA, Barcelona, Spain) and general anesthesia by intraperitoneal (IP) injection (60mg/Kg of Ketamine + 0.25mg/Kg of Dexmedetomidine). In both cohorts animals were re-injected biweekly if IOP measures were less than 20 mmHg in order to keep OHT. At basal time [b]cohort received 3 microliters of a Bri-Lap formulation in right eye by intravitreal injection 1mg/ml brimonidina-Xlaponita using a measured in micrometers Hamilton® syringe and a glass micropipette, which let the visualization of the yellowish formulation being introduced. After intervention animals were let recover under temperature control with warm pads, by enriched oxygen 2.5% atmosphere and lubricant antibiotic ointment on eyes.

Clinical, functional and structural ophthalmological in vivo examination

Ophthalmological clinical signs such as redness, scars, infection or intraocular inflammation were evaluated every week; as well as measurements of intraocular pressure (IOP) recorded with the rebound tonometer Tonolab®. The value of the IOP was the average of three consecutive measurements, which resulted from the average of 6 rebounds. For that purpose rats were sedated for less than three minutes with a mixture of 3% sevoflurane gas and 1.5% oxygen to avoid the potential effect of gas anesthesia, as recommended (Chun Ding et al 2011).
Functionality of neurorretinal structures were studied with electroretinography (ERG) (Roland consult® RETIanimal ERG, Germany) by Flash scotopic ERG and Photopic Negative Response (PhNR) protocols at baseline, 8, 12 and 24 week. For testing Flash scotopic ERG, animals were previously dark adapted for 12 hours in a dark room, anesthetized with intraperitoneal (IP) and topical anesthesia and fully dilated with mydriatics eye drops (Tropicamide 10mg/ml, Phenylephrine 100mg/ml, Alcon Cusí® SA, Barcelona, Spain) and cornea lubricated (Hypromellose 2%). Corneal electrodes served as active electrodes, reference electrodes were placed subcutaneously at both sides and the ground electrode nearby its tail. Electrode impedance was accepted if there was a difference $<2\Omega$ between electrodes. Both eyes were simultaneously tested by a Ganzfeld Q450 SC sphere with white LEDs flashes for stimuli and these seven steps with increasing intensity of luminance and intervals were performed (step 1: 0.0003 cds/m$^2$, 0.2Hz/s; steep 2: 0.003 cds/m$^2$, 0.125Hz/s; step 3: 0.03 cds/m$^2$, 8.929Hz/s; step 4: 0.03 cds/m$^2$, 0.111Hz/s; step 5:0.3 cds/m$^2$, 0.077Hz/s; step 6: 3.0 cds/m$^2$, 0.067Hz/s; and step 7: 3.0 cds/m$^2$, 29.412Hz/s). Then PhNR protocol was performed after light adaptation to blue background (470 nm, 25 cds/m$^2$) and red LED flash (625nm, cds/m$^2$) was used as stimuli. Latency (in milliseconds) and amplitude (in microvolts) were studied in a, b and PhNR waves.
Neurorretinal structures were studied with optical coherence tomography (OCT Spectralis, Heidelberg® Engineering, Germany) at baseline, third day and at 2, 4, 6, 8, 12, 24 weeks after the Bri-Lap injection. Protocols such as retina posterior pole (R), retina nerve fiber layer (RNFL) and ganglion cells layer (GCL) with automatic segmentation were used. These protocols analyzed an area of 1, 2 and 3 mm around the center of the optic disc by X scans and subsequent follow-up examinations were acquired at this same location using the eye tracking software and follow-up application. Vitreous was also visualized. For the acquisition of scans, rats were IP anesthetized (as it was said before) and a contact lens was adapted on its cornea to get images with high quality.
Biased examinations were discarded or manually corrected by a masked trained technician if the algorithm had obviously erred.
Animals were killed under humanity conditions with an intracardiac injection of sodium tiopenthal (25mg/ml) under general anesthesia and eyes were immediately enucleated for histological studies.

**Statistical analysis:**

This was a longitudinal and interventionist study. All data were registered in a Excel database, and statistical analysis was performed using SPSS software version 20.0 (SPSS Inc., Chicago, IL). The Kolmogorov-Smirnov test was used to assess sample distribution. Given the non-parametric distribution of the most data, the differences between both cohorts were evaluated using the Mann-Whitney U test and the changes registered in each eye over the 24 weeks study period were compared using a paired Wilcoson test. All values were expressed as means $\pm$ standard deviations. Values of p <0.05 were considered to indicate statistical significance. To avoid a high false-positive rate, the Bonferroni correction for multiple comparisons was calculated. The level of significance for each variable was established based on Bonferroni calculations.

**RESULTS**

**IOP and clinical signs**

[a]cohort: OD with OHT inducted vs OS as control eye.

A raised of IOP >20mmHg in OD was found since first week after OHT induction until week 10 reaching the peak value (29.92$\pm$7.39 mmHg) at week 7. Since week 11 to the end of the study (week 24) IOP maintained a plateau level ranged between 17.38$\pm$2.87 to 23.66$\pm$5.45 mmHg (Figure 3a).

[b]cohort: OD with OHT inducted + intravitreal Bri-Lap formulation injected vs OS with OHT inducted.

After the induction of OHT and the intravitreal injection of Bri-Lap formulation at baseline, OD showed levels of IOP <20mmHg (10.35±2.50 to 14.96±4.16 mmHg) until week 3, since then IOP was constant and ranged between 17.36±4.10 to 23.99±4.04 mmHg until the end of the study. OS also showed progressive increased of IOP throughout 24 weeks of follow-up, but the IOP in OD was higher than in OS over the 24 weeks. Statistically significant differences were found at day 3 (12.29±2.02 vs 11.24±1.97mmHg, p=0.007*), week 1 (10.36±1.21 vs 9.72±1.44mmHg, p=0.029), week 3 (20.05±4.48 vs 17.86±3.98mmHg, p=0.037), week 7 (21.24±4.07 vs 16.33±3.27mmHg, p=0.002*), week 16 (21.79±1.42 vs 18.13±3.94mmHg, p=0.047) and week 17 (19.33±2.73 vs 14.79±2.43mmHg, p=0.047) (Figure 3b).

When OD from [a]cohort and [b]cohort were compared, the right eyes treated with Bri-Lap ([b]cohort) always experienced lower IOP levels and statistical differences were found from week 1 to 10, but even Bonferroni correction for multiple comparisons(*) was exceeded from week 1 to 8 (p<0.001*). However, this trend inverted from week 12 and not statistical differences were found since then. So, Bri-Lap intravitreal formulation statistically diminished IOP measurements until week 10, later differences between cohorts were not found. The percentage of OHT (>20 mmHg) eyes in both cohorts was studied and the analysis revealed a lower percentage of hypertensive right eyes treated with Bri-Lap from [b]cohort compared to the OHT but no treated right eyes from [a]cohort up to week 8. This was especially remarkable during the first month (0% vs 72% at week 1, 4.8% vs 88% at week 2 or 28.1% vs 91.7% at week 4, respectively) but this tendency was inverted later. Nevertheless, a higher percentage of hypertensive eyes treated with Bri-Lap intravitreal formulation from [b]cohort, in relation to its left eyes used as hypertensive control eyes was found throughout the study (Figure 3c).

On the other side, when OS from [a]cohort (no intervened eyes but with OHT inducted in its contralateral eye) vs OS from [b]cohort (OHT eyes without treatment but with Bri-Lap treatment in its OHT contralateral eye) were compared, a lower percentage of eyes with OHT was found in those from [b]cohort until week 8. This showed that initiate Bri-Lap treatment in an eye could also control the IOP of its contralateral eye at early times.

None case of infection, intraocular inflammation or retinal detachment was found. Two animals developed cataract formation while the episcleral vein sclerosis was being performed, but it reverted spontaneously the subsequent weeks. One case of cataract formation after the intravitreal injection was developed, probably due to surgical issues as rats have thick lens. As a remarkable adverse event, fifteen early and non-expected death animals were found with any obvious cause; concretely four rats at week 2, four rats at week 4, six rats at week 8 and one rat at week 12.

## ERG

OD from [a]cohort showed tendency of loss of amplitude at week 24 respects to week 12 in waves: a (13.25±16.78 vs 67.53±138.31 μV), b (44.64±28.20 vs 56.54±53.34 μV) and PhNR (18.68±24.77 vs 25.52±26.79 μV) explored by PhNR protocol, but no statistical differences were found.

No statistical differences were also found between OD vs OS from [b]cohort according to latency in a and b waves, explored by scotopic flash ERG protocol throughout the study; but higher amplitude was found in OD comparing OS mainly with lower intensity stimulus, and it reached statistically significance for a wave at week 8 in step 1 (39.18±26.04 vs 9.55±7.17 μV, p=0.004*) and step 7 (61.73±46.55 vs 23.64±16.36 μV, p=0.026), at week 12 in step 1 (67.00±21.00 vs 19.66±11.59 μV, p=0.05) and step 4 (33.33±13.05 vs 18.00±2.64 μV, p=0.05) and finally at week 24 in step 2 (32.20±10.68 vs 10.00±8.71 μV, p=0.016) and step 7 (83.20±67.46 vs 8.00±6.24 μV, p=0.028); and for b wave at week 8 in step 1 (73.27±14.77 vs 31.27±16.07 μV, p= 0.000*) and step 7 (99.73±54.58 vs 50.09±27.22 μV, p=0.045), at week 12 in step 1 (85.00±15.39 vs 30.66±15.30 μV, p=0.05), step 3 (211.66±34.48 vs 141.66±7.00 μV, p=0.05) and step 7 (138.33±22.59 vs 57.00±40.36 μV, p=0.05) and finally at week 24 in step 1 (85.60±20.69 vs 35.80±21.47 μV, p=0.009*). When PhNR protocol was performed statistically significant differences were not also found between eyes according to latency; but tendency to its maintenance was observed over 24 weeks. However, statistically significant higher amplitudes in OD vs OS were obtained at week 8 in waves: a (47.18±30.13 vs 18.45±13.09 μV, p=0.018*), b (83.27±31.97 vs 36.82±14.89 μV, p=0.001*) and PhNR (58.45±30.95 vs 28.09±16.34 μV, p=0.012*); at week 12 in waves: a (32.33±7.76 vs 8.33±2.08 μV, p=0.05), b (59.66±22.14 vs 22.66±9.07 μV, p=0.05) and PhNR (76.00±36.29 vs 23.33±13.50 μV, p=0.05) and finally at week 24 in waves b (73.80±40.35 vs 27.80±3.03 μV, p=0.011*) and PhNR (41.20±8.75 vs 14.60±2.60 μV, p=0.015*). Furthermore a progressive increasing amplitudes trend in PhNR wave, from week 8 to week 12, was found in OD (Figure 4).

When OD from [a] vs [b] cohorts were compared no statistically differences were found according to latency using any protocol. Scotopic ERG test at week 24, showed statistically significant worse results in amplitude and latency parameters in [b]cohort; but PhNR protocol revealed statistically significant higher amplitudes in eyes treated with Bri-Lap formulation at week 12 in PhNR wave (25.52±26.79 vs 76.00±36.29 μV, p=0.032) and at week 24 in a wave (13.25±16.78 vs 58.20±49.82 μV, p=0.028) and PhNR (18.68±24.77 vs 41.20±8.75 μV, p=0.042). These results suggested that Bri-Lap intravitreal formulation did not exert protective effect on photoreceptors' functionality in late periods, but it did on retinal ganglion cells (Figure 5).

**OCT**

OD from [a]cohort showed a progressive loss in R, RNFL and GCL thickness measured by OCT over 24 weeks of follow up.

Analyzing the entire retinal thickness, OD from [b]cohort showed a thicker thickness trend and lower percentage loss, explored with Retina protocol up to week 8 compared to its contralateral OS hypertensive no-treated eye; and statistically differences were found in inner sectors, specifically at day 3 in inner nasal (272.20±10.68 vs 257.80±7.12 $\mu$m, p=0.03), at week 2 in inner inferior (262.85±18.42 vs 254.36±10.48 $\mu$m, p=0.02*), at week 6 in central (295.00±10.29 vs 269.60±11.86 $\mu$m, p=0.01*) and in inner superior (264.40±6.95 vs 248.60±4.43 $\mu$m, p=0.04) sectors. OD also experienced an increased thickness in all retinal sectors at day 3 and week 4, compared to their previous exploration. In ulterior explorations (weeks 12 and 24) no statistical differences were found between right and left eyes. These results suggested a protective effect of Bri-Lap intravitreal formulation over retina structure in early times, up to week 8; even when IOP of right eyes was statistically significant higher than their contralateral left eyes.

According to RNFL protocol, higher thickness and so a lower RNFL percentage loss, was found in OD over every time of follow up; but statistically significant differences were just found at early periods such as at day 3 in nasal superior sector (49.20±7.69 vs 35.60±6.73 $\mu$m, p=0.01*); at week 2 in temporal inferior (51.56±15.04 vs 44.53±8.76, $\mu$m p=0.029), temporal (53.19±12.20 vs 46.19±11.26 $\mu$m, p=0.01*) and global (49.66±11.63 vs 44.56±5.53 $\mu$m, p=0.02*) sectors; at week 4 in temporal inferior sector (52.05±11.24 vs 43.19±8.73 $\mu$m, p=0.00*); and at week 6 in temporal superior (64.40±22.78 vs 40.00±9.05 $\mu$m, p=0.05) and nasal superior (45.80±7.46 vs 28.25±6.23 $\mu$m, p=0.01*) sectors. A striking increase in thickness in most sectors was also found at day 3. These results showed an early protective effect on the axonal structure in eyes treated with Bri-Lap, even when IOP was statistically significant higher than their contralateral no-treated left eyes, and this was the trend until the end of the study.

When analyzing the GCL protocol, a higher GCL thickness, and a lower GCL percentage loss, was found in early times in OS and it was statistically significant in outer sectors, specifically at week 2 in outer nasal sector (22.06±3.11 vs 23.75±3.11, p=0.02*) and at week 4 in outer temporal sector (23.24±2.87 vs 25.07±2.12, p=0.03). However, a lower percentage loss trend in OD was observed throughout the rest of the follow-up.

Neuro-retina examinations using OCT technology showed that OD with intravitreal Bri-Lap formulation experienced statistically significant lower percentage thickness loss and so an enhanced structural protection in early/intermediate times up to week 6 and week 8 regarding to RNFL and Retina respectively; but GCL showed this tendency at intermediate (weeks 6 and 8) and late (weeks 12 and 24) times compared to OS.

With the aim to find out the total effect exerted by Bri-Lap, OD from [a] and [b] cohorts were compared . Retina scans from [b]cohort showed tendency to thicker thickness at early times and it was statistically significant at week 6 in the central (265.05±19.43 vs 295.00±10.29 $\mu$m, p=0.003) and inner superior (255.18±15.56 vs 264.40±6.95 $\mu$m, p=0.031) sectors; but later explorations, thicker retina thickness was found in eyes without treatment and it was statistically significant at week 8 in outer superior (264.71±21.46 vs 246.25±12.95 $\mu$m, p=0.042) and outer temporal (270.00±30.82 vs 247.78±.71 $\mu$m, p=0.039) sectors and at week 24 in outer nasal sector (250.00±8.22 vs 240.40±4.66 $\mu$m, p=0.028). Analyzing the RNFL protocol, a tendency to thicker thickness in those eyes injected with Bri-Lap was observed from early/intermediate times until the end of the study; and statistically significant differences were found at week 6 in nasal superior (32.55±14.37 vs 45.80±.46, p=0.039), temporal superior (37.81±15.72 vs 64.40±22.78 $\mu$m, p=0.007*), and temporal (46.68±10.46 vs 67.20±26.16 $\mu$m, p=0.029) sectors, at week 8 in temporal inferior (41.17±5.07 vs 54.43±13.60 $\mu$m, p=0.032), nasal inferior (44.50±6.53vs56.25±9.45 $\mu$m, p=0.028) sectors and at week 24 in temporal inferior (38.29±4.46 vs 45.40±5.81 $\mu$m, p=0.034) sector. According to GCL examinations at week 6, all sectors had higher thicknesses but temporal sector in [b]cohort, with statistically significance in total volume (0.12±0.07 vs 0.8±0.01 $\mu$m, p=0.039) central (17.45±3.15 vs 25.00±4.52 $\mu$m, p=0.002*), inner inferior (23.41±4.42 vs 29.80±2.16 $\mu$m, p=0.002*) and inner superior (22.59±3.37 vs 27.20±2.38 $\mu$m, p=0.009*) sectors; at week 8 the tendency inverted and [a]cohort had thicker thickness, but again at week 12 [b]cohort showed an upward trend toward higher thickness and finally at week 24 every single sector from group injected with Bri-Lap had higher thickness and it was statistically significant in inner inferior (21.57±5.59 vs 25.60±1.67 $\mu$m, p=0.038) and outer temporal (20.43±5.28 vs 24.80±1.78 $\mu$m, p=0.024) sectors.

These results demonstrated that Bri-Lap formulation had a protective effect over the structure of Retina at early times (weeks 0 to 6); as well as axonal and ganglionar at intermediate and late times (weeks 6 to 24) if it was intravitreally injected in an eye with OHT.

To evaluate if Bri-Lap formulation could exert some kind of effect on the contralateral eye, OS from [a]cohort (control eye without any intervention on it, but with contralateral OHT eye) vs OS from [b]cohort (OHT eye without treatment BUT with Bri-Lap injection in its contralateral OHT eye) were compared. No statistical differences were found in most sectors in all the times that were analyzed, except at week 24, when a higher RNFL thickness was measured in [b]cohort explored by RNFL protocol in nasal superior (18.57±6.24 vs 34.60±10.55 μm, p=0.023) sector and in GCL thickness explored by GCL protocol in total volume (0.14±0.01 vs 0.15±0.01 μm, p=0.03) and inner inferior sector (21.71±1.60 vs 26.20±1.09 μm, p=0.004*). OS from both cohorts were >20mmHg at week 24, however at that moment OS from [b]cohort had statistically higher axonal and ganglionar thickness than OS from [a]cohort. So Bri-Lap intravitreal formulation exerted axonal and ganglionar protection in the injected eye from week 6, but also in its contralateral non-injected eye at week 24, and thickness measurements were even higher than those from eyes no noxa inducted.

Images from vitreous scans showed hyper reflective aggregates of Bri-Lap formulation dispersed in the vitreous gel as floaters, with tendency to go to the vitreo-retinal interface, and by OCT-guided evidence of crossing it and contacting to the retinal tissue. A progressive decreased in the number and size of Bri-Lap aggregates was also detected by OCT over time, as a method to monitor its degradation.

**Claims**

1. A composition for the controlled delivery of non-ionic drugs, wherein the composition comprises laponite and a non-ionic drug and wherein the laponite acts as a carrier for the controlled delivery of the non-ionic drug, with the proviso that said composition does not comprise polyurethane.

2. The composition of claim 1, wherein the composition is in the form of a powder.

3. The composition of claim 1, wherein the composition is a homogenous dispersion or suspension suitable to be inyected intravitreously or suprachoroidally.

4. The composition of claim 3, wherein the ratio of the non-ionic drug and the laponite present in said composition is from 1:3 to 1:25, preferably between 1:5 and 1:15.

5. The composition of any of claims 1 to 4, wherein the non-ionic drug is selected from the group consisting of a corticosteroid component such as cortisone, prednisolone, triamcinolone, triamcinolone acetonide, fluorometholone, dexamethasone, medrysone, loteprednol, derivatives thereof and mixtures thereof, or selective alpha-adrenergic receptor agonists such as Brimonidine.

6. A pharmaceutical composition suitable for treating a posterior ocular condition, comprising the composition of any of claims 1 to 5.

7. The pharmaceutical composition of claim 6, wherein the pharmaceutical composition comprises dexamethasone or Brimonidine.

8. The composition of any of claims 1 to 5 or the pharmaceutical composition of claims 6 or 7, for use in a method for treating a posterior ocular condition, the method comprising the step of administering the composition to the vitreous of a human or animal, thereby treating the posterior ocular condition.

9. The composition for use according to claim 8, wherein the administering step comprises intravitreal injecting.

10. The composition for use according to any of claims 8 to 9, wherein the method is for treating open-angle glaucoma, or ocular hypertension, the composition comprising brimonidina, and the method comprising the step of administering to the vitreous of a human eye the composition.

11. The composition for use according to any of claims 8 to 9, wherein the method is for treating chronic ocular diseases, the composition comprises a corticosteroid component such as cortisone, prednesolone, triamcinolone, triamcinolone acetonide, fluorometholone, dexamethosone, medrysone, loteprednol, and the method comprising the step of administering to the vitreous of a human eye the composition.

12. The composition for use according to the previous claim, wherein the corticosteroid component is dexamethasone.

13. The composition for use according to any of claims 8 to 9, or 11 to 12, wherein the method is for treating maculopathies/retinal degeneration, uveitis/retinitis/choroiditis, Vascular diseases/exudative diseases, traumatic/surgical diseases; proliferative disorders, genetic disorders and tumors.

**14.** A method of preparing the composition of claim 3, the method comprising:

> a. Dissolving the drug in a hydrophobic medium, such as acetone, and then adding LAP to it;
> b. Stirring the product of step a), preferably at room temperature for about 1 hour, and then removing the solvent, preferably in a rotary evaporator; and
> c. Drying the product obtained from step b) to form a powder.

**15.** A method of preparing the composition of any of claims 1 and 3 to 5, the method comprising:

> a. Dissolving the drug in a hydrophobic medium, such as acetone, and then adding LAP to it;
> b. Stirring the product of step a), preferably at room temperature for about 1 hour, and then removing the solvent, preferably in a rotary evaporator;
> c. Drying the product obtained from step b); and
> d. Prior to injection, the dried product obtained in step c must be suspended in a suitable aqueous medium to yield a transparent and homogeneous dispersion.

Fig. 1.

Fig. 2.

Fig. 3a.

Fig 3b.

Fig. 3c.

OHT eyes (%)

Legend:
- OD [a]cohort EPIm
- OD [b]cohort EPIm+BL
- OS [a]cohort EPIm without sclerosis
- OS [b]cohort EPIm+BL without BL

Time

EP 3 851 097 A1

22

A)

Fig. 4.

Fig. 4 (Cont.)

A)

Fig. 5.

Fig. 5 (Cont.)

B)

PhNR ERG a,b,g[µV]

EP 3 851 097 A1

# EP 3 851 097 A1

**EUROPEAN SEARCH REPORT**

Application Number

EP 20 38 2021

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JOSÉ M. FRAILE ET AL: "Laponite as carrier for controlled in vitro delivery of dexamethasone in vitreous humor models", EUROPEAN JOURNAL OF PHARMACEUTICS AND BIOPHARMACEUTICS, vol. 108, 1 November 2016 (2016-11-01), pages 83-90, XP055712497, NL ISSN: 0939-6411, DOI: 10.1016/j.ejpb.2016.08.015 * abstract * * paragraph [0001] * * paragraph [02.1] - paragraph [02.3] * * paragraph [03.2]; figures 6-9 * * paragraph [0004] * | 1,2,4-9, 11-15 | INV. A61K9/10 A61K9/14 A61K47/02 A61K31/498 A61K31/573 A61K9/00 |
| X | M J RODRIGO ET AL: "T109 Laponite clay for long term delivery dexamethasone intravitreal injections", ACTA OPHTHAMOLOGICA (ONLINE), vol. 96, no. S261, 12 December 2018 (2018-12-12), page 106, XP055712504, Denmark, United Kingdom, United States, Netherlands ISSN: 1755-3768 * the whole document * | 1,3-9, 11-13 | |
| | | | TECHNICAL FIELDS SEARCHED (IPC) |
| | | | A61K |
| X | WO 03/059194 A2 (ALCON INC [CH]; KETELSON HOWARD ALLEN [US]; MEADOWS DAVID L [US]) 24 July 2003 (2003-07-24) * page 4, line 1 - line 15 * * page 6, line 7 - line 15 * * page 7, line 19 - page 8, line 14 * * page 8, line 16 - page 9, line 2 * * examples * * claims * | 1,3-6 | |

-/--

The present search report has been drawn up for all claims

| Place of search | Date of completion of the search | Examiner |
|---|---|---|
| The Hague | 9 July 2020 | Epskamp, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

27

Europäisches
Patentamt

European
Patent Office

Office européen
des brevets

**EUROPEAN SEARCH REPORT**

Application Number

EP 20 38 2021

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/070402 A2 (ALLERGAN INC [US]; ROBINSON MICHAEL R [US] ET AL.) 12 June 2008 (2008-06-12) * claims 10-25 * * examples 2-7 * * page 9, line 11 - line 15 * * page 12, line 1 - line 13 * ----- | 1,8-10 | |

TECHNICAL FIELDS
SEARCHED      (IPC)

The present search report has been drawn up for all claims

| Place of search | Date of completion of the search | Examiner |
|---|---|---|
| The Hague | 9 July 2020 | Epskamp, Stefan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.......................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.                    EP 20 38 2021

This annex lists the patent family members relating to the patent documents cited in the above-mentioned European search report.
The members are as contained in the European Patent Office EDP file on
The European Patent Office is in no way liable for these particulars which are merely given for the purpose of information.

09-07-2020

| Patent document cited in search report | | Publication date | Patent family member(s) | | Publication date |
|---|---|---|---|---|---|
| WO 03059194 | A2 | 24-07-2003 | AT | 478657 T | 15-09-2010 |
| | | | AU | 2002367029 A1 | 30-07-2003 |
| | | | BR | 0215238 A | 31-05-2005 |
| | | | CA | 2467763 A1 | 24-07-2003 |
| | | | CN | 1604770 A | 06-04-2005 |
| | | | DK | 1474109 T3 | 25-10-2010 |
| | | | EP | 1474109 A2 | 10-11-2004 |
| | | | ES | 2349235 T3 | 29-12-2010 |
| | | | HK | 1075001 A1 | 24-07-2009 |
| | | | JP | 2005514429 A | 19-05-2005 |
| | | | KR | 20040072669 A | 18-08-2004 |
| | | | MX | PA04004919 A | 11-08-2004 |
| | | | PL | 211494 B1 | 31-05-2012 |
| | | | PT | 1474109 E | 25-10-2010 |
| | | | SI | 1474109 T1 | 30-11-2010 |
| | | | US | 2005003014 A1 | 06-01-2005 |
| | | | US | 2011274760 A1 | 10-11-2011 |
| | | | WO | 03059194 A2 | 24-07-2003 |
| | | | ZA | 200403666 B | 27-07-2005 |
| WO 2008070402 | A2 | 12-06-2008 | AR | 064078 A1 | 11-03-2009 |
| | | | AU | 2007329723 A1 | 12-06-2008 |
| | | | BR | PI0721055 A2 | 29-07-2014 |
| | | | CA | 2670746 A1 | 12-06-2008 |
| | | | CL | 2007003464 A1 | 25-09-2009 |
| | | | CL | 2011003220 A1 | 22-06-2012 |
| | | | CN | 101588770 A | 25-11-2009 |
| | | | CN | 102512356 A | 27-06-2012 |
| | | | CN | 103893106 A | 02-07-2014 |
| | | | EP | 2099387 A2 | 16-09-2009 |
| | | | EP | 2218424 A1 | 18-08-2010 |
| | | | IL | 198804 A | 31-03-2014 |
| | | | JP | 5419703 B2 | 19-02-2014 |
| | | | JP | 2010511433 A | 15-04-2010 |
| | | | JP | 2014128580 A | 10-07-2014 |
| | | | KR | 20090085154 A | 06-08-2009 |
| | | | KR | 20140084252 A | 04-07-2014 |
| | | | NZ | 577105 A | 26-10-2012 |
| | | | NZ | 600921 A | 30-05-2014 |
| | | | NZ | 619297 A | 31-07-2015 |
| | | | RU | 2009122647 A | 10-01-2011 |
| | | | TW | 200826943 A | 01-07-2008 |
| | | | TW | 201438718 A | 16-10-2014 |
| | | | US | 2008131484 A1 | 05-06-2008 |
| | | | US | 2014341968 A1 | 20-11-2014 |
| | | | WO | 2008070402 A2 | 12-06-2008 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**

EP 20 38 2021

This annex lists the patent family members relating to the patent documents cited in the above-mentioned European search report.
The members are as contained in the European Patent Office EDP file on
The European Patent Office is in no way liable for these particulars which are merely given for the purpose of information.

09-07-2020

| Patent document cited in search report | Publication date | Patent family member(s) | Publication date |
|---|---|---|---|
| ------------------------------------------------------------------------------- | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DA SILVA GR ; AYRES E ; OREFICE RL ; MOURA SA ; CARA DC ; CUNHA ADA S JR.** Controlled release of dexamethasone acetate from biodegradable and biocompatible polyurethane and polyurethane nanocomposite. *J Drug Target,* 2009, vol. 17, 374-383

- **HERRERA et al.** *J. Mater. Chem.,* 2005, 863-871
- **CUMMINS, J.** *Non-Crystalline Solids,* 2007, vol. 353, 3891-3905